# EUROPEAN PATENT APPLICATION

(11) **EP 1 918 275 A1**
(43) Date of publication of application: **07.05.2008**
(21) Application number: 07254349.9
(22) Date of filing: 02.11.2007
(51) Int. Cl.: C07D 207/16, C07D 409/12, C07F 9/09, C07C 229/36

(54) **Production method of diphenylalanine - NI(II) complex**

(30) Priority: 03.11.2006 US 856289 P
(71) Applicant: Ajinomoto Co., Inc., Tokyo 104-8315 (JP)
(72) Inventor: Hamada, Takayuki c/o Ajinomoto Co., Inc., Kawasaki-shi, Kanagawa 210-8681 (JP); Izawa, Kunisuke c/o Ajinomoto Co., Inc., Kawasaki-shi, Kanagawa 210-8681 (JP); Soloshonok, Vadim A. c/o Ajinomoto Co., Inc., Kawasaki-shi, Kanagawa 210-8681 (JP)
(74) Representative: Nicholls, Kathryn Margaret

(57) **Abstract**

The present invention relates to a production method of a compound represented by the formula (3), which includes reacting a compound represented by the formula (2) with a diphenylmethyl halide compound represented by the formula (1) in the presence of alkali metal alkoxide. According to the method of the present invention, compound (3) can be produced at a high purity and in a high yield. 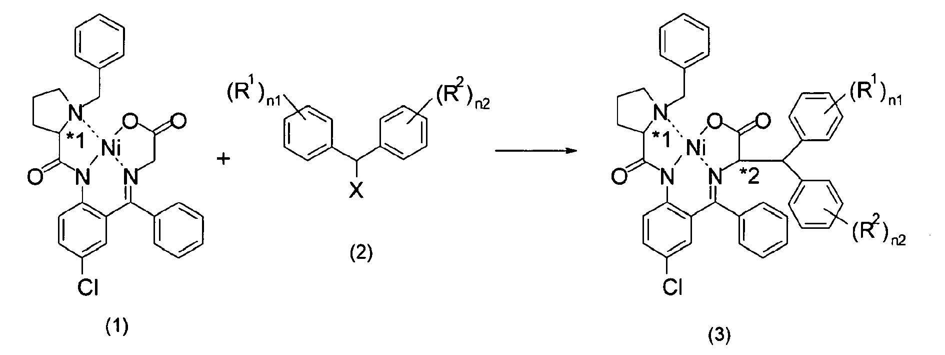 wherein each symbol is as defined in the specification, *1 and *2 each show an asymmetric carbon atom, and the configuration of the asymmetric carbon atom of *1 and *2 is (S,S) or (R,R).

## Description

The present invention relates to a production method of a diphenylalanine-Ni(II) complex useful as a synthetic intermediate for an optically active diphenylalanine compound, and a production method of an optically active diphenylalanine compound.

A diphenylalanine compound, particularly an optically active diphenylalanine compound is useful as an intermediate for pharmaceutical agents and has been used, for example, as an intermediate for anti-HIV drugs (WO04/056764, U.S. Patent No. 6,632,816 and U.S. Patent Application Publication No. 2006/0025592), thrombin inhibitors (WO00/39124 and U.S. Patent No. 6,492,402) or dipeptidyl peptidase inhibitors (WO03/002531 and U.S. Patent Application Publication Nos. 2004/0167341, 2004/0171848 and 2004/0242636).

As a production method of optically active diphenylalanine, a method using (S)- or (R)-2-[(N-benzylprolyl)amino]benzophenone (hereinafter to be also referred to as BPB) is known (Tetrahedron: Asymmetry, Vol. 8, No. 1, pp. 79-83, 1997). According to this method, as shown in the following scheme, an Ni(II) complex of Schiff's base obtained from glycine and (S)-BPB (hereinafter to be also referred to as Ni-(S)-BPB-Gly) is reacted with diphenylmethyl chloride in acetonitrile and in the presence of sodium hydride, and the obtained diphenylalanine-Ni (II) complex (hereinafter to be also referred to as Ni-BPB-DIP) is treated with an acid to stereoselectively give (S)-diphenylalanine. By a similar reaction using (R)-BPB, (R)-diphenylalanine can be stereoselectively produced.

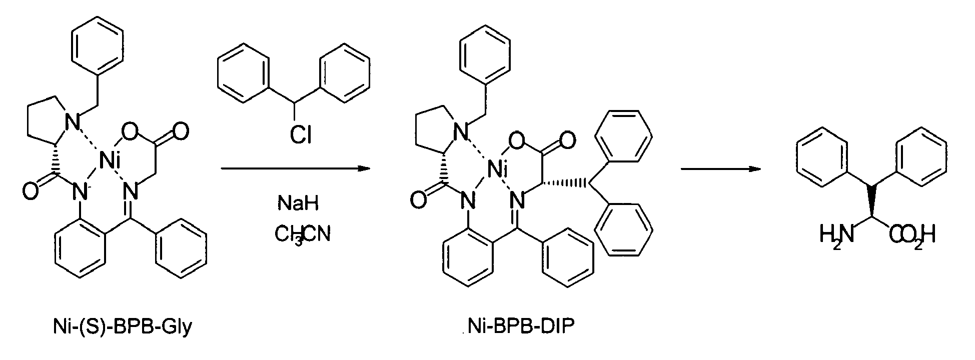

According to the above-mentioned method, the diastereomeric excess of Ni-BPB-DIP obtained from Ni-(S)-BPB-Gly is 90%d.e., which is insufficient in the stereoselectivity. When diphenylmethyl chloride is used as a substrate, problems occur in that sodium hydride used as a base is difficult to handle since it is pyrophoric, and a low temperature reactor is used at -78°C, which is unsuitable for industrial production.

It is therefore an object of the present invention to provide a method of producing a diphenylalanine-Ni(II) complex at a high purity and in a high yield, which is suitable for industrial production.

The present inventors have conducted intensive studies in an attempt to solve the above-mentioned problems and found that the diastereoselectivity of the asymmetric alkylation reaction for the production of the optically active diphenylalanine compound can be improved, e.g. from 90%d.e. to 96%d.e., and that a method for suitable for industrial production can be provided, by changing the ligand from optically active BPB to optically active 2-[(N-benzylprolyl)amino]-5-chlorobenzophenone (hereinafter to be also referred to as BPC), and the use of alkali metal alkoxide as a base, which resulted in the completion of the present invention. 96%d.e. on the diastereoselectivity means 2% of isomer content, therefore purification is easy compared to 5% of isomer content for 90%d.e. compound and a high purity object compound can be obtained in a high yield.

Accordingly, the present invention provides the following.
[1] A method for producing a compound represented by the formula (3) 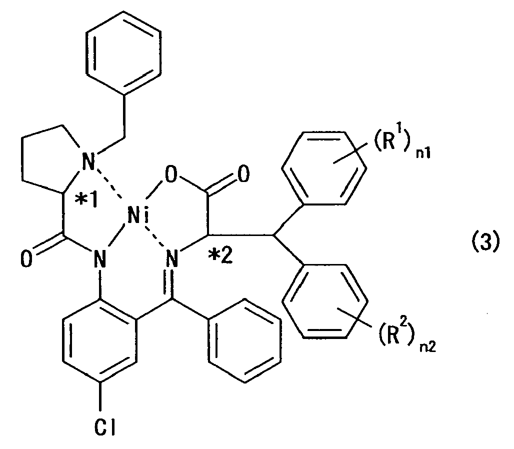 wherein R¹ and R² are each independently a halogen atom, a substituted or unsubstituted alkyl group, a substituted or unsubstituted alkoxy group, a substituted or unsubstituted amino group, a nitro group, a hydroxyl group or a protected hydroxyl group, n1 and n2 are each independently an integer of 0 to 5, *1 and *2 show an asymmetric carbon atom, and the configuration of the asymmetric carbon atom of *1 and *2 is (S,S) or (R,R), which comprises reacting a compound represented by the formula (1) 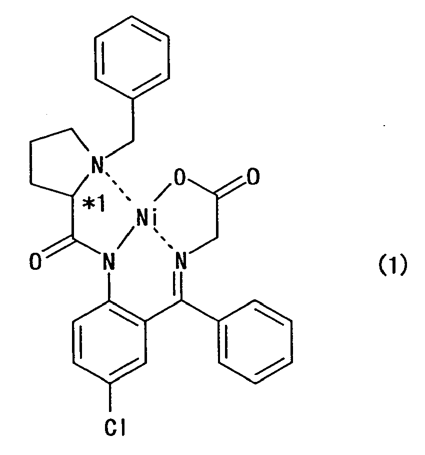 wherein *1 shows an asymmetric carbon atom, and the configuration of the asymmetric carbon atom is S or R, with a diphenylmethyl halide compound represented by the formula (2) 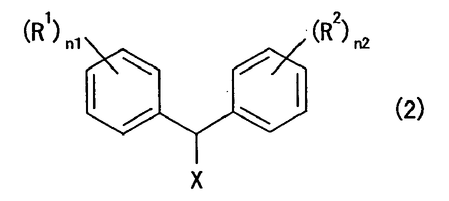 wherein R¹, R², n1 and n2 are as defined above, and X is a halogen atom, in the presence of alkali metal alkoxide.
[2] The method of the above-mentioned [1], wherein the alkali metal alkoxide is selected from the group consisting of sodium methoxide, sodium ethoxide, sodium isopropoxide, sodium tert-butoxide and potassium tert-butoxide.
[3] The method of the above-mentioned [1], wherein the alkali metal alkoxide is sodium methoxide.
[4] The method of any one of the above-mentioned [1]-[3], wherein X is a chlorine atom, a bromine atom or an iodine atom.
[5] The method of any one of the above-mentioned [1]-[3], wherein X is a chlorine atom.
[6] The method of any one of the above-mentioned [1]-[5], wherein n1 and n2 are each 0 or 1.
[7] The method of any one of the above-mentioned [1]-[6], wherein the configuration of the asymmetric carbon atom of *1 is S and the configuration of the asymmetric carbon atom of *2 is S.
[8] The method of any one of the above-mentioned [1]-[6], wherein the configuration of the asymmetric carbon atom of *1 is R and the configuration of the asymmetric carbon atom of *2 is R.
[9] A method for producing an optically active diphenylalanine compound represented by the formula (4) 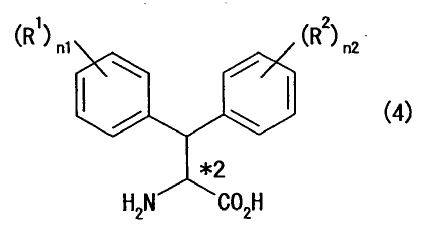 wherein R¹, R², n1 and n2 are as defined in the above-mentioned [1], *2 shows an asymmetric carbon atom, and the configuration of the asymmetric carbon atom is S or R, or a salt thereof, which comprises obtaining a compound represented by the formula (3) according to the method of any one of the above-mentioned [1]-[5], and treating the compound of the formula (3) with an acid.
[10] The method of the above-mentioned [9], wherein the acid is selected from the group consisting of hydrochloric acid, hydrobromic acid, sulfuric acid, nitric acid, acetic acid and trifluoroacetic acid.
[11] The method of the above-mentioned [9] or [10], wherein n1 and n2 are each 0 or 1.
[12] The method of any one of the above-mentioned [9]-[11], wherein the configuration of the asymmetric carbon atom of *1 is S and the configuration of the asymmetric carbon atom of *2 is S.
[13] The method of any one of the above-mentioned [9]-[11], wherein the configuration of the asymmetric carbon atom of *1 is R and the configuration of the asymmetric carbon atom of *2 is R.
[14] A compound represented by the formula (3) 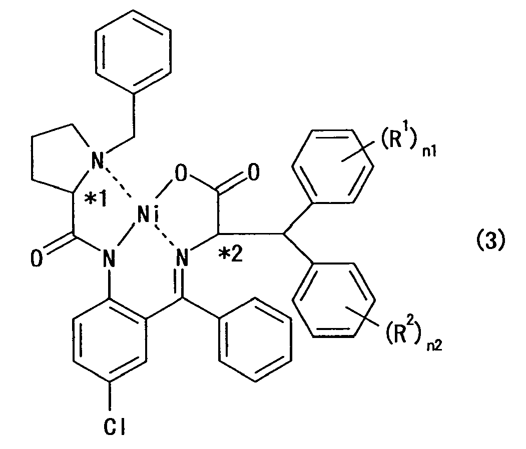 wherein R¹ and R² are each independently a halogen atom, a substituted or unsubstituted alkyl group, a substituted or unsubstituted alkoxy group, a substituted or unsubstituted amino group, a nitro group, a hydroxyl group or a protected hydroxyl group, n1 and n2 are each independently an integer of 0 to 5, *1 and *2 show an asymmetric carbon atom, and the configuration of the asymmetric carbon atom of *1 and *2 is (S,S) or (R,R).
[15] In a method for preparing a compound of the formula (I) 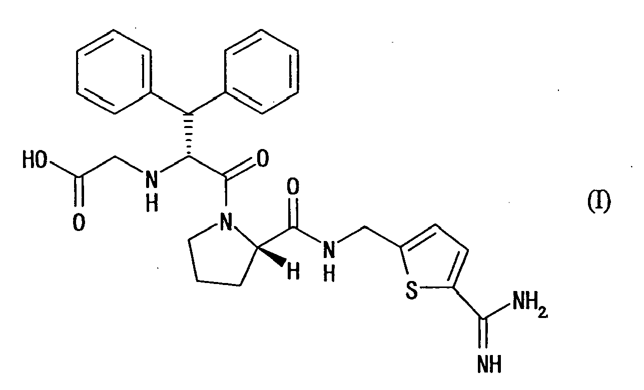 or a salt thereof,
   said method comprising converting a compound of the formula (II) 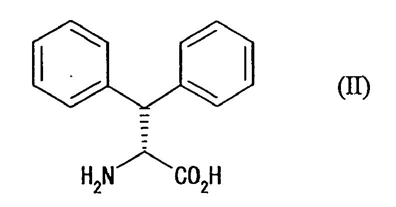 or a salt thereof into said compound of the formula (I) or a salt thereof, the improvement being said compound of the formula (II) or a salt thereof is prepared by a method according to the above-mentioned [9].
[16] In a method for preparing a compound of the formula (III) 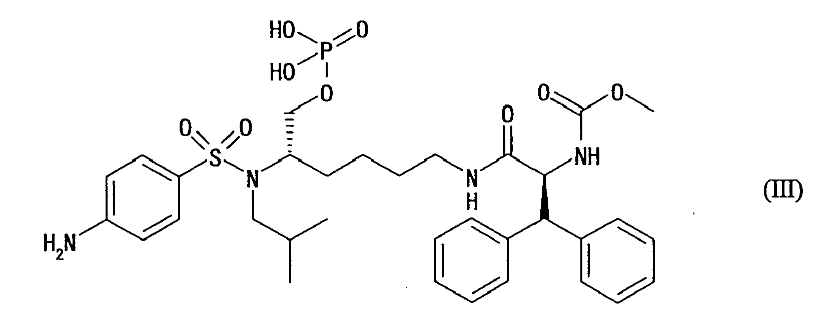 or a salt thereof,
   said method comprising converting a compound of the formula (IV) 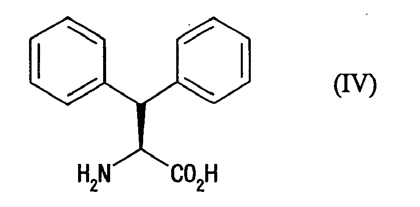 or a salt thereof into said compound of the formula (III) or a salt thereof, the improvement being said compound of the formula (IV) or a salt thereof is prepared by a method according to the above-mentioned [9].
[17] In a method for preparing a compound of the formula (V) 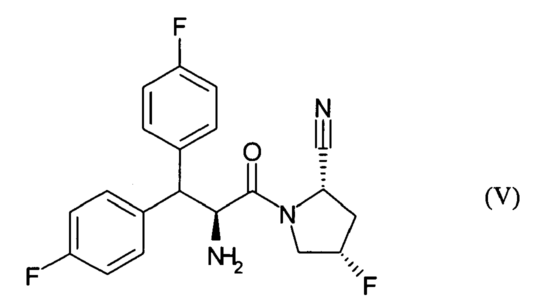 or a salt thereof,
   said method comprising converting a compound of the formula (VI) 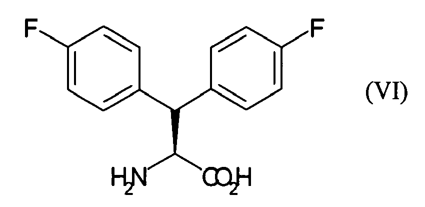 or a salt thereof into said compound of the formula (V) or a salt thereof, the improvement being said compound of the formula (VI) or a salt thereof is prepared by a method according to the above-mentioned [9].

The method of the present invention is a production method advantageous in the following points.

According to the method of the present invention, the diastereomer selectivity on production of a compound of the formula (3) can be improved and the compound can be produced at a high purity and in a high yield. Furthermore, since alkali metal alkoxide, not highly dangerous sodium hydride, is used as a base and the reaction can be carried out at ambient temperature, the method is suitable for industrial production.

Embodiments of the present invention are described in detail in the following.

The definitions of the symbols used in the respective formulas in the present specification are first explained.

As the halogen atom for X, a chlorine atom, a bromine atom and an iodine atom are preferable, a chlorine atom and a bromine atom are more preferable, and a chlorine atom is most preferable.

As the halogen atom for R¹ or R², a fluorine atom, a chlorine atom, a bromine atom and an iodine atom can be mentioned.

As the alkyl group for R¹ or R², a linear or branched alkyl group having preferably 1-10 carbon atoms, more preferably 1-7 carbon atoms, still more preferably 1-4 carbon atoms, is preferable. To be specific, methyl group, ethyl group, propyl group, isopropyl group, butyl group, isobutyl group, sec-butyl group, tert-butyl group, pentyl group, hexyl group, heptyl group, octyl group and the like can be mentioned. Of these, methyl group, ethyl group, propyl group, isopropyl group, butyl group, tert-butyl group and the like are preferable. The alkyl group is optionally substituted by one or more substituents such as a halogen atom (e.g., chlorine atom, bromine atom, fluorine atom), a hydroxyl group, an alkoxy group having 1-6 carbon atoms (e.g., methoxy group) and the like.

As the alkoxy group for R¹ or R², a linear or branched alkoxy group having preferably 1-10 carbon atoms, more preferably 1-7 carbon atoms, still more preferably 1-4 carbon atoms, is preferable. To be specific, methoxy group, ethoxy group, propoxy group, isopropoxy group, butoxy group, isobutoxy group, sec-butoxy group, tert-butoxy group, pentyloxy group, hexyloxy group, heptyloxy group, octyloxy group and the like can be mentioned. Of these, methoxy group, ethoxy group, propoxy group, isopropoxy group, butoxy group, tert-butoxy group and the like are preferable. The alkoxy group is optionally substituted by one or more substituents such as a halogen atom (e.g., chlorine atom, bromine atom, fluorine atom), a hydroxyl group, an alkoxy group having 1-6 carbon atoms (e.g., methoxy group) and the like.

The amino group for R¹ or R² is optionally mono- or disubstituted by the aforementioned alkyl group, aryl group or aralkyl group, or optionally protected by a group exemplified for the below-mentioned amino-protecting group. As the aryl group, an aryl group having 6-14 (preferably 6-8) carbon atoms, such as phenyl group, tolyl group and the like, can be mentioned. The aralkyl group means an alkyl group substituted by an aryl group. The alkyl moiety has preferably 1-6 carbon atoms, more preferably 1-3 carbon atoms. To be specific, methyl group, ethyl group, propyl group, isopropyl group and the like can be mentioned. The aryl moiety has preferably 6-14 (more preferably 6-8) carbon atoms. To be specific, phenyl group, naphthyl group and the like can be mentioned. The total carbon number of the aralkyl group is preferably 7-20, more preferably 7-11. To be specific, benzyl group, 1-phenylethyl group, 2-phenylethyl group and the like can be mentioned. Of these, benzyl group is preferable.

The hydroxyl group for R¹ or R² is optionally protected, and as the protecting group, conventionally-known ones can be mentioned. To be specific, benzyl group, trimethylsilyl group, triethylsilyl group, tert-butyldimethylsilyl group and the like can be mentioned.

When n¹ or n² is an integer of 2 to 5, R¹ in the number of n¹ may be the same or different, and R² in the number of n² may similarly be the same or different.

As the amino-protecting group, the groups described in Protecting Groups in Organic Chemistry 2nd edition (John Wiley & Sons, Inc. 1991) can be mentioned. To be specific, an acyl group, an alkyl group, an aralkyl group, a silyl group and the like can be mentioned. As the acyl group, for example, a formyl group, a C₁₋₆ alkyl-carbonyl group (e.g., acetyl group), a C₆₋₈ aryl-carbonyl group, a C₇₋₁₁ aralkyl-carbonyl group (e.g., phenylacetyl group) and the like can be mentioned. As the alkyl group, for example, those similar to the alkyl group for R¹ or R² can be mentioned. The aralkyl group means an alkyl group substituted by an aryl group. The alkyl moiety has preferably 1-6 carbon atoms, more preferably 1-3 carbon atoms. To be specific, methyl group, ethyl group, propyl group, isopropyl group and the like can be mentioned. The aryl moiety has preferably 6-14 (more preferably 6-8) carbon atoms. To be specific, phenyl group, naphthyl group and the like can be mentioned. The total carbon number of the aralkyl group is preferably 7-20, more preferably 7-11. To be specific, benzyl group, 1-phenylethyl group, 2-phenylethyl group and the like can be mentioned. Of these, benzyl group is preferable. The aralkyl group is optionally substituted by one or more substituents such as a halogen atom (e.g., chlorine atom, bromine atom, fluorine atom), a hydroxyl group, an alkyl group having 1-6 carbon atoms (e.g., methyl group), an alkoxy group having 1-6 carbon atoms (e.g., methoxy group), a haloalkyl group having 1-6 carbon atoms (e.g., trifluoromethyl group), a haloalkoxy group having 1-6 carbon atoms (e.g., trifluoromethoxy group) and the like. As the silyl group, for example, a trialkyl-substituted silyl group such as trimethylsilyl group, triethylsilyl group, tert-butyldimethylsilyl group and the like can be mentioned. The alkyl moiety has preferably 1-4 carbon atoms, and to be specific, methyl group, ethyl group, propyl group, isopropyl group, butyl group, tert-butyl group and the like can be mentioned. In addition, methoxymethyl group, methylthiomethyl group, benzyloxymethyl group, methoxyethoxymethyl group, tetrahydropyranyl group, methoxycarbonyl group (Moc group), 9-fluorenylmethoxycarbonyl group (Fmoc group), 2,2,2-trichloroethoxycarbonyl group, benzyloxycarbonyl group (Cbz group), tert-butoxycarbonyl group (Boc group) and the like can also be mentioned.

As each of R¹ and R², a halogen atom, an alkyl group, having 1-4 carbon atoms, an alkoxy group having 1-4 carbon atoms, an amino group, a nitro group or a hydroxyl group is preferable. R¹ and R² may be the same or different.

Preferably, n¹ and n² are each independently 0, 1 or 2. Particularly preferably, n¹ and n² are each independently 0 or 1.

Now, the production method of the present invention is explained below. The production method of the present invention is shown in the following reaction scheme. 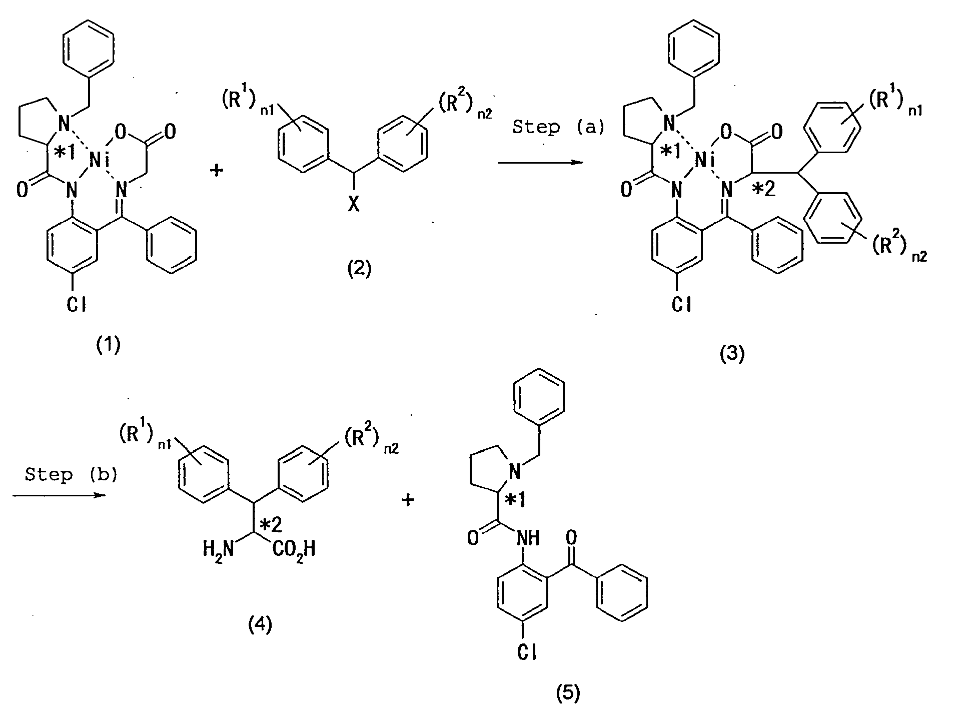 In the above-mentioned scheme, each symbol is as defined above.

### Step (a)

In Step (a), a compound represented by the formula (1) (hereinafter to be referred to as compound (1)) is reacted with a diphenylmethyl halide compound represented by the formula (2) (hereinafter to be referred to as compound (2)) in the presence of alkali metal alkoxide to give a compound represented by the formula (3) (hereinafter to be referred to as compound (3)).

Using a compound of the formula (1), wherein the configuration of the asymmetric carbon atom is S, in this step, a compound of the formula (3), wherein the configuration of the asymmetric carbon atom of *1 and *2 is (S,S) can be stereoselectively obtained. Similarly, using a compound of the formula (1), wherein the configuration of the asymmetric carbon atom is R, a compound of the formula (3), wherein the configuration of the asymmetric carbon atom of *1 and *2 is (R,R) can be stereoselectively obtained.

The amount of compound (2) to be used is generally 0.8 to 2.0 mol, preferably 0.9 to 1.2 mol, per 1 mol of compound (1).

As the alkali metal alkoxide, alkali metal C₁-C₄ alkoxide such as sodium methoxide, sodium ethoxide, sodium isopropoxide, sodium tert-butoxide, potassium tert-butoxide and the like are preferable, and sodium methoxide is more preferable.

The amount of the alkali metal alkoxide to be used is generally 1 to 2 mol, preferably 1.1 to 1.3 mol, per 1 mol of compound (1).

The alkali metal alkoxide is preferably used as a solution of the same alcohol as the alcohol moiety of the alkoxide. For example, when sodium methoxide is used, it is preferably used as a methanol solution, and when sodium ethoxide is used, it is preferably used as an ethanol solution.

The reaction in this step is preferably carried out in a solvent that does not inhibit the reaction. As such solvent, amide solvents (e.g., N,N-dimethylformamide, N,N-dimethylacetamide, N-methyl-2-pyrrolidone, N-ethyl-2-pyrrolidone, etc.), sulfoxide solvents (e.g., dimethyl sulfoxide, etc.), nitrile solvents (e.g., acetonitrile, etc.), ether solvents (e.g., diethyl ether, diisopropyl ether, 1,2-dimethoxyethane, tetrahydrofuran, 1,4-dioxane, etc.), alcohol solvents (e.g., methanol, ethanol, isopropanol, etc.) and the like can be mentioned. These solvents may be used in a mixture of two or more kinds thereof in a suitable ratio.

While the amount of the solvent to be used can be appropriately determined according to the kind of the compound, it is generally 1 to 10 L, preferably 2 to 5 L, per 1 kg of compound (1).

While the reaction temperature is not particularly limited, it is generally -20°C to 50°C, preferably 0°C to 30°C. While the reaction time is not particularly limited, it is generally 0.5 to 24 hours, preferably 1 to 8 hours.

The work-up can be performed according to a general method known to those of ordinary skill in the art, and isolation and purification can be performed according to a conventional method appropriately selected as necessary, such as crystallization, recrystallization, distillation, partitioning, silica gel chromatography, preparative HPLC and the like, or according to a combination of these methods.

For example, compound (3) can be obtained by adding, after completion of the reaction, water to the reaction mixture, neutralizing (generally pH 6 to 8, preferably pH 6.5 to 7.5) the mixture with an acid (e.g., aqueous citric acid solution, acetic acid, etc.), and collecting the precipitated solid by filtration.

### Step (b)

In step (b), compound (3) is treated with an acid to give an optically active diphenylalanine compound represented by the formula (4) (hereinafter to be referred to as compound (4)) or a salt thereof.

In this step, using a compound of the formula (3), wherein the configuration of the asymmetric carbon atom of *1 and *2 is (S,S), a compound of the formula (4), wherein the configuration of the asymmetric carbon atom is S can be obtained. Similarly, using a compound of the formula (3), wherein the configuration of the asymmetric carbon atom of *1 and *2 is (R,R), a compound of the formula (4), wherein the configuration of the asymmetric carbon atom is R can be obtained.

As the acid, inorganic acids (e.g., hydrochloric acid, hydrobromic acid, sulfuric acid, nitric acid, etc.) and aqueous solutions thereof, and organic acids (e.g., acetic acid, trifluoroacetic acid, acetyl chloride, thionyl chloride, oxalyl chloride, etc.) can be mentioned. Preferred is hydrochloric acid or sulfuric acid. The amount of the acid to be used is generally 1 to 20 equivalents, preferably 3 to 10 equivalents, per 1 equivalent of compound (3).

The reaction in this step can also be carried out in a solvent that does not inhibit the reaction. As such solvent, water, alcohol solvents (e.g., methanol, ethanol, isopropanol, etc.), ether solvents (e.g., diethyl ether, diisopropyl ether, 1,2-dimethoxyethane, tetrahydrofuran, 1,4-dioxane, etc.), amide solvents (e.g., N,N-dimethylformamide, N,N-dimethylacetamide, N-methyl-2-pyrrolidone, N-ethyl-2-pyrrolidone, etc.), ketone solvents (e.g., acetone, methyl isobutyl ketone, etc.), aromatic hydrocarbon solvents (e.g., benzene, toluene, chlorobenzene, etc.) and the like can be mentioned. These solvents may be used in a mixture of two or more kinds thereof in a suitable ratio. Preferred is methanol. While the amount of the solvent to be used can be appropriately determined according to the kind of the compound, it is generally 3 to 20 L, preferably 5 to 10 L, per 1 kg of compound (3).

While the reaction temperature is not particularly limited, it is generally 25°C to 100°C, preferably 40°C to 70°C. While the reaction time is not particularly limited, it is generally 10 minutes to 10 hours, preferably 30 minutes to 2 hours.

The work-up can be performed according to a general method known to those of ordinary skill in the art, and isolation and purification can be performed according to a conventional method appropriately selected as necessary, such as crystallization, recrystallization, distillation, partitioning, silica gel chromatography, preparative HPLC and the like, or according to a combination of these methods.

The compound represented by the formula (5) or a salt thereof recovered in this step is reacted to in the presence of a base (e.g., sodium methoxide, sodium hydroxide, potassium hydroxide, etc.), with glycine and a salt containing Ni²⁺ (e.g., Ni(NO₃)₂ or hydrate thereof) to give an Ni(II) complex, which can be reused as compound (1).

The compound (1), which is a starting material, can be produced based on a known method (e.g., the method described in J. Org. Chem., Vol. 68, No. 18, pp. 7104-7107, 2003).

Preferably, it can be produced by the following method.

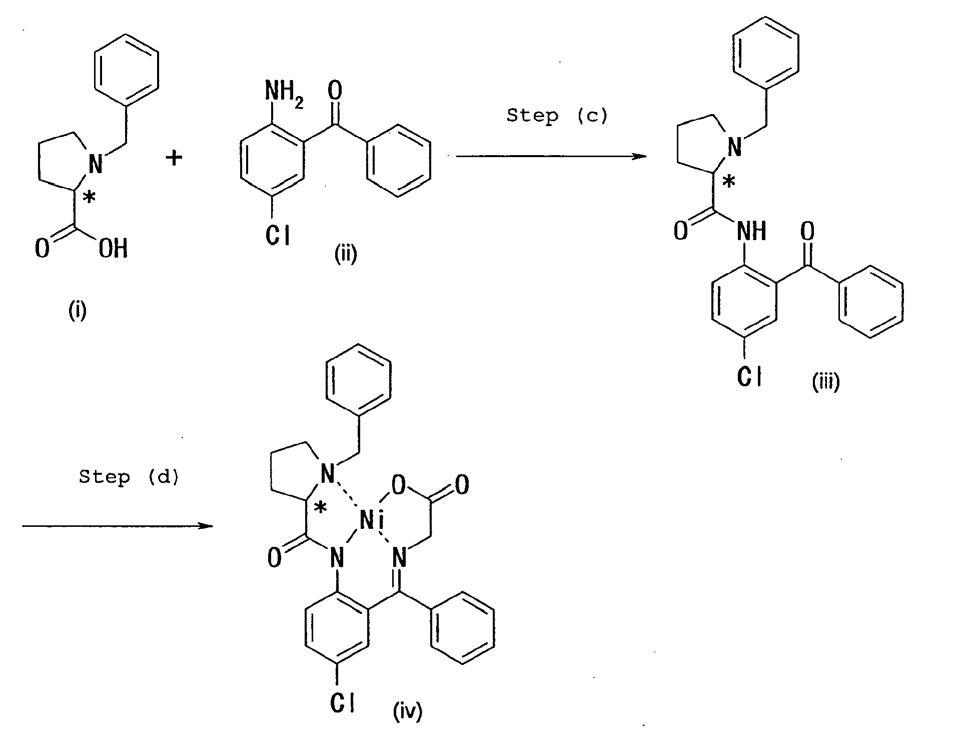

### Step (c)

In Step (c), optically active N-benzylproline represented by the formula (i) (hereinafter to be referred to as compound (i)) is reacted with 2-amino-5-chlorobenzophenone represented by the formula (ii) (hereinafter to be referred to as compound (ii)) in acetonitrile and in the presence of an amide compound and thionyl chloride to give optically active 2-[(N-benzylprolyl)amino]-5-chlorobenzophenone represented by the formula (iii) (hereinafter to be referred to as compound (iii)) or a salt thereof.

The amount of the solvent to be used is generally 3 to 20 L, preferably 5 to 10 L, relative to 1 kg of compound (i). Other solvent may be mixed with acetonitrile as necessary.

As the amide compound, N,N-dialkylformamide such as N,N-dimethylformamide; N,N-dialkylacetamide such as N,N-dimethylacetamide; N-alkyl-2-pyrrolidone such as N-methyl-2-pyrrolidone; and the like can be mentioned. Two or more kinds of these amide compounds may be used in a mixture in a suitable ratio. As the amide compound, N,N-dimethylformamide and N-methyl-2-pyrrolidone are preferable, particularly N,N-dimethylformamide is preferable, from the aspect of yield.

The amount of the amide compound to be used is generally 0.5 to 2 mol, preferably 1.0 to 1.2 mol, per 1 mol of compound (i).

The amount of thionyl chloride to be used is generally 1 to 4 mol, preferably 1.1 to 1.5 mol, per 1 mol of compound (i).

The amount of compound (ii) to be used is generally 0.5 to 2 mol, preferably 0.9 to 1.0 mol, per 1 mol of compound (i).

For the reaction, it is preferable to add thionyl chloride to an acetonitrile solution containing compound (i) and an amide compound and then add compound (ii) to allow a reaction.

While the temperature of the addition of thionyl chloride is not particularly limited, it is generally -78°C to 20°C, preferably -20°C to 10°C. Thionyl chloride is preferably added while stirring the acetonitrile solution, and it is preferable to keep stirring the reaction mixture after the addition of thionyl chloride. While the stirring time after the addition of thionyl chloride is not particularly limited, it is generally 0.1 to 3 hours, preferably 0.5 to 2 hours.

While the reaction temperature of the reaction with compound (ii) is not particularly limited, it is generally -78°C to 25°C, preferably -10°C to 25°C. While the reaction time is not particularly limited, it is generally 1 to 24 hours, preferably 4 to 20 hours.

Since HCl generated in the reaction system and compound (iii) form a hydrochloride, which precipitates as crystals, the hydrochloride of compound (iii) can be easily isolated by filtration of the reaction suspension after completion of the reaction.

The hydrochloride of compound (iii) can be converted to free compound (iii) by treating with a base according to a conventional method. Moreover, the obtained free compound (iii) can be converted to other acid addition salt according to a conventional method.

### Step (d)

In Step (d), compound (iii) or a salt thereof is reacted with glycine and a salt containing Ni²⁺ in the presence of a base to give a compound represented by the formula (iv) (hereinafter to be referred to as compound (iv)).

The amount of glycine to be used is generally 1 to 5 mol, preferably 1.2 to 2.0 mol, per 1 mol of compound (iii) or a salt thereof.

As the salt containing Ni²⁺, nitrate (Ni(NO₃)₂), halide (e.g., NiCl₂, NiBr₂, etc.), acetate (Ni(OAc)₂), sulfate (NiSO₄) and the like can be mentioned. These salts may be a hydrate, and preferably, Ni(NO₃)₂ or a hydrate thereof.

The amount of the salt containing Ni²⁺ is generally 1 to 5 mol, preferably 1.2 to 2.0 mol, per 1 mol of compound (iii) or a salt thereof.

As the base, alkali metal alkoxide (e.g., alkali metal C₁-C₄ alkoxide such as sodium methoxide, sodium ethoxide, sodium isopropoxide, sodium tert-butoxide, potassium tert-butoxide, etc.), alkali metal hydroxide (e.g., sodium hydroxide, potassium hydroxide, etc.) and the like can be mentioned.

The amount of the base to be used is generally 2 to 20 mol, preferably 5 to 10 mol, per 1 mol of compound (iii) or a salt thereof.

The reaction of this step is preferably carried out in a solvent that does not inhibit the reaction. As such solvent, alcohol solvents (e.g., methanol, ethanol, isopropanol, t-butanol, etc.), N,N-dimethylformamide, N,N-dimethylacetamide, N-methyl-2-pyrrolidone and the like can be mentioned.

While the amount of the solvent to be used can be appropriately selected according to the kind of the compound, it is generally 2 to 20 L, preferably 4 to 10 L, relative to 1 kg of compound (iii).

While the reaction temperature is not particularly limited, it is generally 25°C to 100°C, preferably 40°C to 70°C. While the reaction time is not particularly limited, it is generally 10 minutes to 5 hours, preferably 30 minutes to 2 hours.

The work-up can be performed according to a general method known to those of ordinary skill in the art, and isolation and purification can be performed according to a conventional method appropriately selected as necessary, such as crystallization, recrystallization, distillation, partitioning, silica gel chromatography, preparative HPLC and the like, or according to a combination thereof.

According to method of the present invention, using a compound of the formula (i), wherein the configuration of the asymmetric carbon atom is S, compounds of the formula (iii) and the formula (iv), wherein the configuration of the asymmetric carbon atom is S, can be obtained. Similarly, using a compound of the formula (i), wherein the configuration of the asymmetric carbon atom is R, compounds of the formula (iii) and the formula (iv), wherein the configuration of the asymmetric carbon atom is R, can be obtained.

As noted above, the optically active compounds of the formula (4) prepared by the present process are, in turn, useful for preparing anti-HIV drugs, thrombin inhibitors and dipeptidyl peptidase inhibitors. Such methods for preparing anti-HIV drugs, thrombin inhibitors and dipeptidyl peptidase inhibitors are disclosed in, *e.g.,* WO04/056764, U.S. Patent No. 6,632,816, U.S. Patent Application Publication No. 2006/0025592, WO00/39124, U.S. Patent No. 6,492,402, WO03/002531, and U.S. Patent Application Publication Nos. 2004/0167341, 2004/0171848 and 2004/0242636, all of which are incorporated herein by reference in their entireties.

Thus, the present invention also provides a method for preparing a compound of the formula (I) 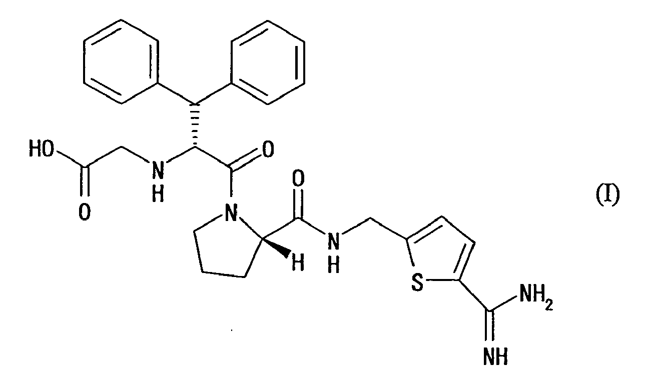 or a salt thereof,
in which a compound of the formula (II) 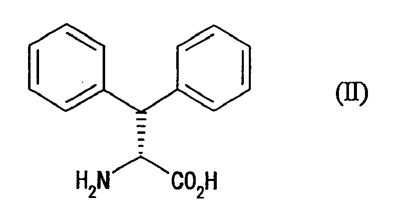 or a salt thereof is converted into the compound of the formula (I) or a salt thereof, and in which the compound of the formula (II) or a salt thereof is prepared by the present method, wherein n1 and n2 are each 0, the configuration of the asymmetric carbon atom of *1 is R, and the configuration of the asymmetric carbon atom of *2 is R.

The compound of the formula (I) or a salt thereof can be prepared according to the method described in WO00/39124 or U.S. Patent No. 6,492,402, from the diphenylalanine compound of the formula (II) or a salt thereof which is prepared by the present method.

The present invention further provides a method for preparing a compound of the formula (III) 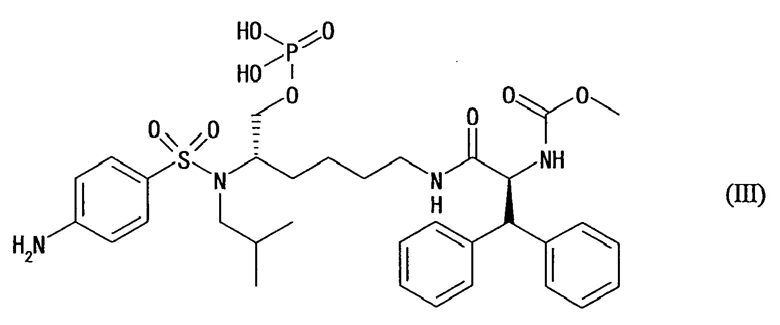 or a salt thereof,
in which a compound of the formula (IV) 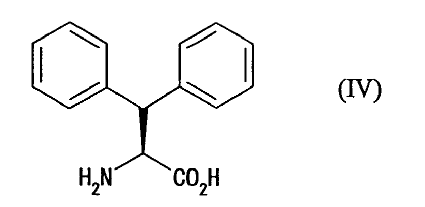 or a salt thereof is converted into the compound of the formula (III) or a salt thereof, and in which the compound of the formula (IV) or a salt thereof is prepared by the present method, wherein n1 and n2 are each 0, the configuration of the asymmetric carbon atom of *1 is S, and the configuration of the asymmetric carbon atom of *2 is S.

The compound of the formula (III) or a salt thereof can be prepared according to the method described in U.S. Patent Application Publication No. 2006/0025592, from the diphenylalanine compound of the formula (IV) or a salt thereof which is prepared by the present method.

The present invention further provides a method for preparing a compound of the formula (V) 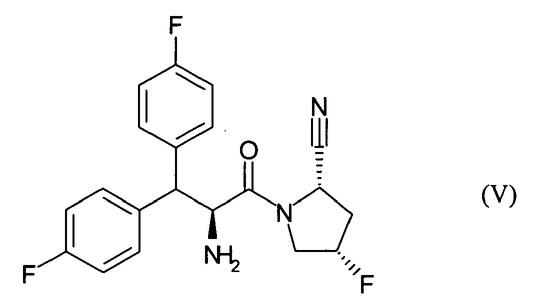 or a salt thereof,
in which a compound of the formula (VI) 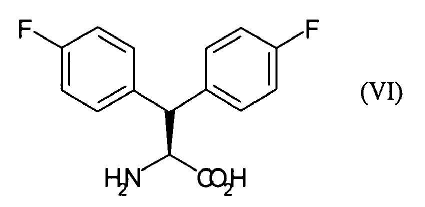 or a salt thereof is converted into the compound of the formula (V) or a salt thereof, and in which the compound of the formula (VI) or a salt thereof is prepared by the present method, wherein n1 and n2 are each 1, R¹ and R² are each 4-fluoro, the configuration of the asymmetric carbon atom of *1 is S, and the configuration of the asymmetric carbon atom of *2 is S.

The compound of the formula (V) or a salt thereof can be prepared according to the method described in WO03/002531 or U.S. Patent Application Publication No. 2004/0171848, from the diphenylalanine compound of the formula (VI) or a salt thereof which is prepared by the present method.

### EXAMPLES

Embodiments of the present invention are explained in more detail in the following by referring to Examples, which are not to be construed as limitative.

In the Examples, the diastereomeric excess (d.e.) of compound (3) was measured under the following conditions.

### <HPLC conditions>

| | |
|---|---|
| Column: | Inertsil ODS-3 (4.6 x 250 mm) |
| Eluent: | A:B= 100:0 to 0:100 (0 to 20 min) |
| | A:B= 0:100 (20 to 30 min) |
| | A:B= 0:100 (30 to 40 min) |
| | A=0.03M KH₂PO₄ aq:CH₃CN=90:10 |
| | B=0.03M KH₂PO₄ aq:CH₃CN=25:75 |
| Flow rate: | 1.0 mL/ min |
| Temp.: | room temperature |
| Detector: | 210 nm |
| Retention time: | Ni-(S)-BPC-(S)-DIP 28 min |
| | Ni-(S)-BPC-(R)-DIP 33 min |

In Reference Example 1, the purity and the enantiomeric excess (e.e.) of (S)-2-[(N-benzylprolyl)amino]-5-chlorobenzophenone hydrochloride were measured under the following conditions.

### <HPLC conditions-purity>

| | |
|---|---|
| Column: | Inertsil ODS-3 (4.6 x 250 mm) |
| Eluent: | A:B= 100:0 to 0:100 (0 to 20 min) |
| | A:B= 0:100 (20 to 30 min) |
| | A:B= 0:100 (30 to 40 min) |
| | A=0.03M KH₂PO₄ aq:CH₃CN=90:10 |
| | B=0.03M KH₂PO₄ aq:CH₃CN=25:75 |
| Flow rate: | 1.0 mL/min |
| Temp.: | room temperature |
| Detector: | 210 nm |

### <HPLC conditions-chiral>

| | |
|---|---|
| Column: | CHIRALPAK AD (4.6 x 250 mm) |
| Eluent: | hexane:isopropanol=1:1 |
| Flow rate: | 1.0 mL/min |
| Temp.: | room temperature |
| Detector: | 220 nm |
| Retention time: | (R)-BPC-HCl 5.4 min |
| | (S)-BPC-HCl 6.3 min |

### Reference Example 1

To a solution of N-benzyl-L-proline (2.05 g, 10 mmol) in acetonitrile (20 mL) was added N,N-dimethylformamide (775 mL, 10 mmol) and the mixture was cooled to -10°C. Thionyl chloride (0.875 mL, 12 mmol) was slowly added dropwise, and the mixture was stirred for 1 hour. Then, 2-amino-5-chlorobenzophenone (2.09 g, 9 mmol) was added, the temperature was raised to 25°C, and the mixture was stirred for 16 hours. After completion of the reaction, the reaction suspension was filtered, and the filtrated crystals were washed with acetone (3 mL) and vacuum dried to give (S)-2-[(N-benzylprolyl)amino]-5-chlorobenzophenone hydrochloride as white crystals (3.15 g, yield 76%, purity not less than 99%, enantiomeric excess not less than 99%e.e.).

### Reference Example 2

To a solution of nickel nitrate hexahydrate (6.96 g, 23.9 mmol) in methanol (20 mL) were added (S)-2-[(N-benzylprolyl)amino]-5-chlorobenzophenone hydrochloride (9.10 g, 20.0 mmol) and glycine (2.25 g, 30.0 mmol), and the mixture was heated to 50°C. 28% sodium methoxide/methanol solution (22 mL) was added, and the mixture was stirred at 60°C for 1 hour. After completion of the reaction, the reaction mixture was added to water to give a reaction suspension. The suspension was filtered and the filtrated solid was vacuum dried to give nickel-(S)-2-[(N-benzylprolyl)amino]-5-chlorobenzophenone-glycine complex as brown crystals (9.76 g, yield 92%).

### Example 1

### Production of Ni-(S)-2-[(N-benzylprolyl)amino]-5-chlorobenzophenone-(S)-diphenylalanine complex (Ni-(S)-BPC-(S)-DIP)

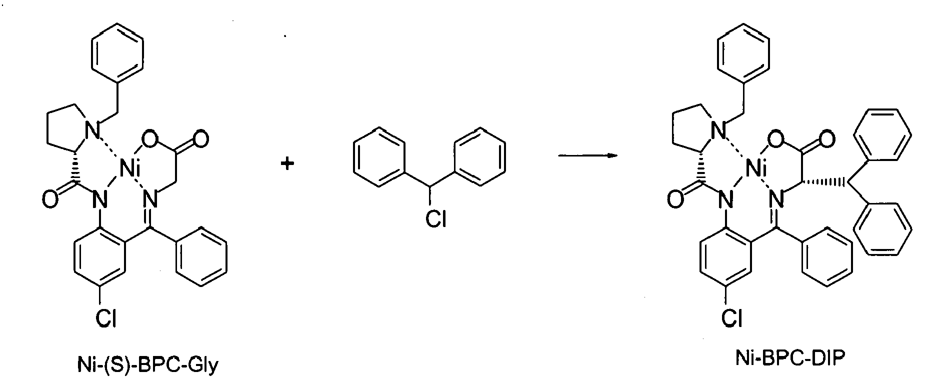

To a solution of Ni-(S)-2-[(N-benzylprolyl)amino]-5-chlorobenzophenone-glycine complex (Ni-(S)-BPC-Gly) (532 mg, 1.0 mmol) in N,N-dimethylformamide (1.25 mL) was added diphenylmethyl chloride (117 µL, 1.0 mmol), and the mixture was stirred at 25°C, a solution (245 µL, 1.2 mmol) of 28% sodium methoxide in methanol was added dropwise, and the mixture was stirred for 6 hours. After completion of the reaction, the reaction mixture was quenched with water, adjusted to pH 7.0 with aqueous citric acid solution, and the suspension was filtered. The filtrated solid was washed with water and vacuum dried to give Ni-BPC-DIP as red crystals (642 mg, yield 92%). The diastereomeric excess of Ni-(S)-BPC-(S)-DIP was 96%d.e.

### Reference Example 3

In the same manner as in Example 1 except that Ni-(S)-2-[(N-benzylprolyl)amino]benzophenone-glycine complex (Ni-(S)-BPB-Gly) was used instead of Ni-(S)-BPC-Gly, Ni-(S)-2-[(N-benzylprolyl)amino]benzophenone-(S)-diphenylalanine complex (Ni-(S)-BPB-(S)-DIP) was produced. The diastereomeric excess of Ni-(S)-BPB-(S)-DIP was 90%d.e.

According to the present invention, a method for producing compound (3) at a high purity and in a high yield, which is suitable for industrial production can be provided. By treating compound (3) with an acid, an optically active diphenylalanine compound useful as a synthetic intermediate for pharmaceutical products can be stereoselectively produced.

Obviously, numerous modifications and variations of the present invention are possible in light of the above teachings. It is therefore to be understood that, within the scope of the appended claims, the invention may be practiced otherwise than as specifically described herein.

All patents, patent application publications and other references mentioned above are incorporated in full herein by this reference, the same as if set forth at length.

## Claims

1. A method for producing a compound represented by the formula (3) 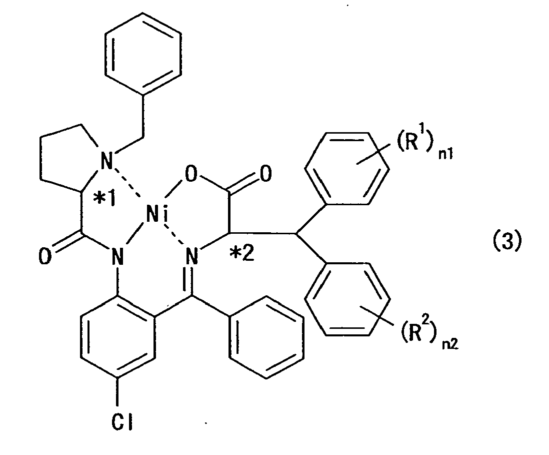 wherein R¹ and R² are each independently a halogen atom, a substituted or unsubstituted alkyl group, a substituted or unsubstituted alkoxy group, a substituted or unsubstituted amino group, a nitro group, a hydroxyl group or a protected hydroxyl group, n1 and n2 are each independently an integer of 0 to 5, *1 and *2 show an asymmetric carbon atom, and the configuration of the asymmetric carbon atom of *1 and *2 is (S,S) or (R,R), which comprises reacting a compound represented by the formula (1) 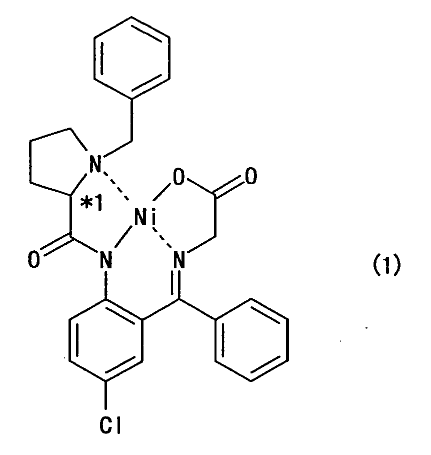 wherein *1 shows an asymmetric carbon atom, and the configuration of the asymmetric carbon atom is S or R, with a diphenylmethyl halide compound represented by the formula (2) 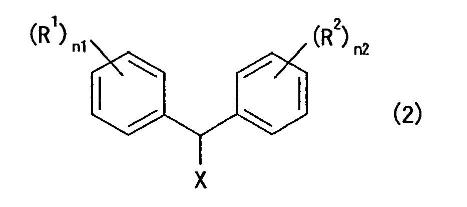 wherein R¹, R², n1 and n2 are as defined above, and X is a halogen atom, in the presence of alkali metal alkoxide.

2. The method of claim 1, wherein the alkali metal alkoxide is selected from the group consisting of sodium methoxide, sodium ethoxide, sodium isopropoxide, sodium tert-butoxide and potassium tert-butoxide.

3. The method of claim 1, wherein the alkali metal alkoxide is sodium methoxide.

4. The method of any one of claims 1-3, wherein X is a chlorine atom, a bromine atom or an iodine atom.

5. The method of any one of claims 1-3, wherein X is a chlorine atom.

6. The method of any one of claims 1-5, wherein n1 and n2 are each 0 or 1.

7. The method of any one of claims 1-6, wherein the configuration of the asymmetric carbon atom of *1 is S and the configuration of the asymmetric carbon atom of *2 is S.

8. The method of any one of claims 1-6, wherein the configuration of the asymmetric carbon atom of *1 is R and the configuration of the asymmetric carbon atom of *2 is R.

9. A method for producing an optically active diphenylalanine compound represented by the formula (4) 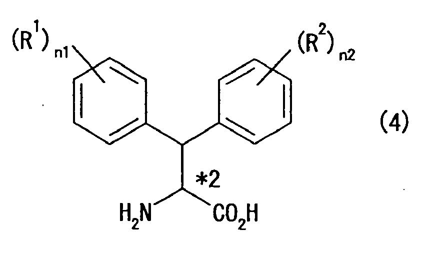 wherein R¹, R², n1 and n2 are as defined in claim 1, *2 shows an asymmetric carbon atom, and the configuration of the asymmetric carbon atom is S or R, or a salt thereof, which comprises obtaining a compound represented by the formula (3) according to the method of any one of claims 1-5, and treating the compound of the formula (3) with an acid.

10. The method of claim 9, wherein the acid is selected from the group consisting of hydrochloric acid, hydrobromic acid, sulfuric acid, nitric acid, acetic acid and trifluoroacetic acid.

11. The method of claim 9 or 10, wherein n1 and n2 are each 0 or 1.

12. The method of any one of claims 9-11, wherein the configuration of the asymmetric carbon atom of *1 is S and the configuration of the asymmetric carbon atom of *2 is S.

13. The method of any one of claims 9-11, wherein the configuration of the asymmetric carbon atom of *1 is R and the configuration of the asymmetric carbon atom of *2 is R.

14. A compound represented by the formula (3) 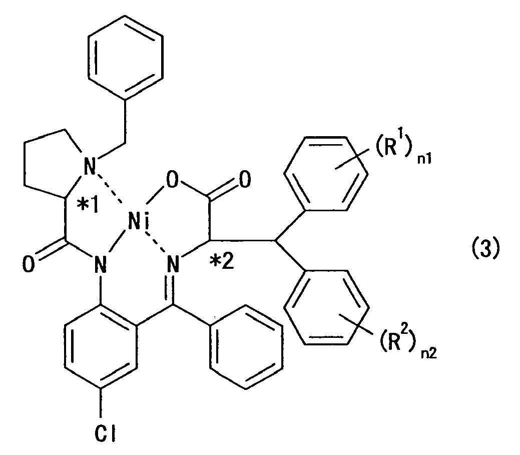 wherein R¹ and R² are each independently a halogen atom, a substituted or unsubstituted alkyl group, a substituted or unsubstituted alkoxy group, a substituted or unsubstituted amino group, a nitro group, a hydroxyl group or a protected hydroxyl group, n1 and n2 are each independently an integer of 0 to 5, *1 and *2 show an asymmetric carbon atom, and the configuration of the asymmetric carbon atom of *1 and *2 is (S,S) or (R,R).

15. In a method for preparing a compound of the formula (I) 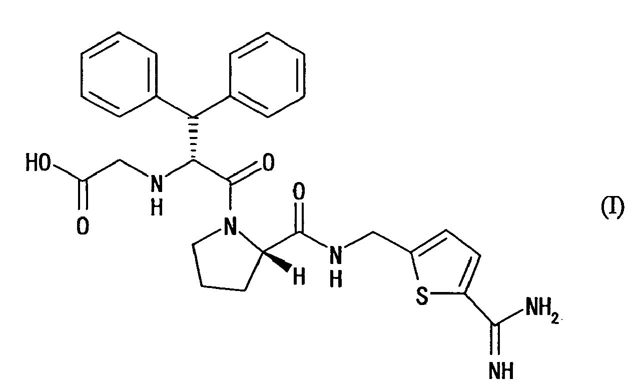 or a salt thereof,
said method comprising converting a compound of the formula (II) 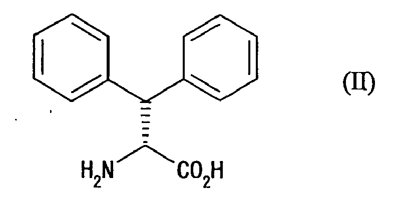 or a salt thereof into said compound of the formula (I) or a salt thereof, the improvement being said compound of the formula (II) or a salt thereof is prepared by a method according to claim 9.

16. In a method for preparing a compound of the formula (III) 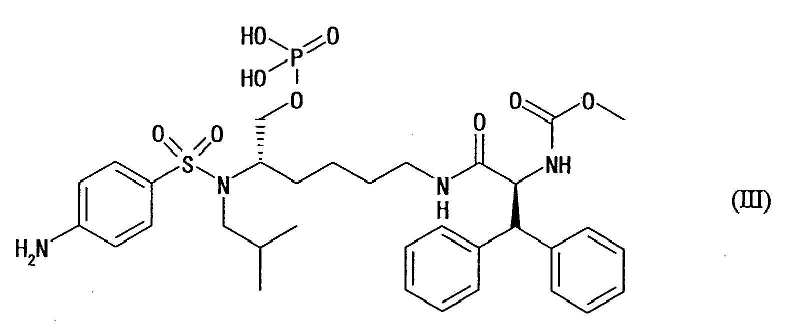 or a salt thereof,
said method comprising converting a compound of the formula (IV) 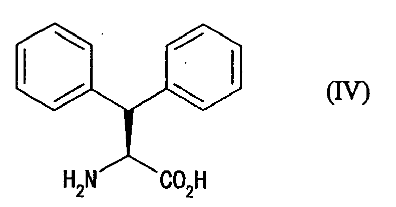 or a salt thereof into said compound of the formula (III) or a salt thereof, the improvement being said compound of the formula (IV) or a salt thereof is prepared by a method according to claim 9.

17. In a method for preparing a compound of the formula (V) 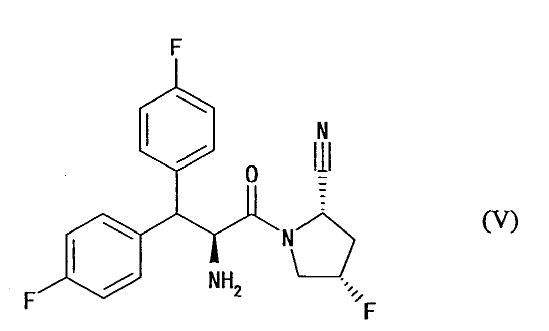 or a salt thereof,
said method comprising converting a compound of the formula (VI) 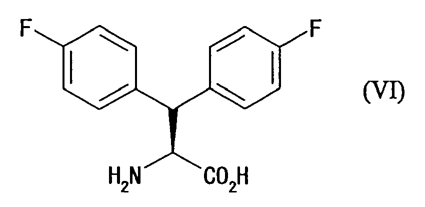 or a salt thereof into said compound of the formula (V) or a salt thereof, the improvement being said compound of the formula (VI) or a salt thereof is prepared by a method according to claim 9.
